(19)

Europäisches
Patentamt
European
Patent Office
Office européen
des brevets

(11) **EP 2 856 371 B1**

(12) **EUROPEAN PATENT SPECIFICATION**

(45) Date of publication and mention
of the grant of the patent:
**09.10.2019 Bulletin 2019/41**

(51) Int Cl.:
*G16H 20/40* (2018.01)   *G16H 50/30* (2018.01)
*G16H 50/20* (2018.01)

(21) Application number: **13735419.7**

(22) Date of filing: **14.05.2013**

(86) International application number:
**PCT/IB2013/053915**

(87) International publication number:
**WO 2013/179170 (05.12.2013 Gazette 2013/49)**

(54) **METHOD AND SYSTEM FOR SELECTING THE FREQUENCY OF ARTERIAL BLOOD GAS TESTING FOR NEONATES**

VERFAHREN UND SYSTEM ZUR AUSWAHL DER FREQUENZ VON ARTERIENBLUTGASTESTS FÜR NEUGEBORENE

PROCÉDÉ ET SYSTÈME DE SÉLECTION DE LA FRÉQUENCE D'UN TEST DE GAZOMÉTRIE DU SANG ARTÉRIEL POUR NOUVEAUX-NÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.06.2012 US 201261654226 P**

(43) Date of publication of application:
**08.04.2015 Bulletin 2015/15**

(73) Proprietor: **Koninklijke Philips N.V.**
**5656 AE Eindhoven (NL)**

(72) Inventor: **MAKKAPATI, Vishnu Vardhan**
**5656 AE Eindhoven (NL)**

(74) Representative: **Steffen, Thomas**
**Philips Intellectual Property & Standards**
**High Tech Campus 5**
**5656 AE Eindhoven (NL)**

(56) References cited:
• **PAOLO MERLANI ET AL: "Quality Improvement Report - Linking guideline to regular feedback to increase appropriate requests for clinical tests: blood gas analysis in intensive care", BMJ - BRITISH MEDICAL JOURNAL, vol. 323, 15 September 2001 (2001-09-15), pages 620-624, XP055094150,**

• **THOMAS J WANG ET AL: "A utilization management intervention to reduce unnecessary testing in the coronary care unit", ARCHIVES OF INTERNAL MEDICINE, vol. 162, no. 16, 9 September 2002 (2002-09-09), pages 1885-1890, XP055094427, ISSN: 0003-9926, DOI: 10.1001/archinte.162.16.1885**

• **DAVID J DURAND ET AL: "CHAPTER 18: Blood Gases: Technical Aspects and Interpretation", 2011, ASSISTED VENTILATION OF THE NEONATE (FIFTH EDITION), ELSEVIER, PAGE(S) 292 - 305, XP009175236, ISBN: 978-1-4160-5624-9 cited in the application section Choice of Monitoring Methods; page 299**

• **P BANSAL ET AL: "A computer based intervention on the appropriate use of arterial blood gas", PROCEEDINGS AMIA SYMPOSIUM, 3 November 2001 (2001-11-03), - 7 November 2001 (2001-11-07), pages 32-36, XP055094355, United States ISSN: 1531-605X**

• **"AARC Clinical Practice Guideline Sampling for Arterial Blood Gas Analysis", RESPIRATORY CARE, vol. 37, August 1992 (1992-08), pages 891-897, XP055094017,**

• **ROBERT T BROUILLETTE ET AL: "Evaluation of the newborn's blood gas status", CLINICAL CHEMISTRY, vol. 43, no. 1, January 1997 (1997-01), page 215, XP055094473, ISSN: 0009-9147**

- R. N SHIFFMAN ET AL: "Computer-based Guideline Implementation Systems: A Systematic Review of Functionality and Effectiveness", JOURNAL OF THE AMERICAN MEDICAL INFORMATICS ASSOCIATION, vol. 6, no. 2, March 1999 (1999-03), pages 104-114, XP055094590, ISSN: 1067-5027, DOI: 10.1136/jamia.1999.0060104

## Description

Neonates suffering from severe respiratory stress are typically put on a ventilator and are regularly monitored to assess changes in their clinical condition. "Quality Improvement Report - Linking guideline to regular feedback to increase appropriate requests for clinical tests: blood gas analysis in intensive care", 15 September 2001, by Paolo Merlani et al, discloses an assessment of the impact of a multifaceted intervention aimed at changing requests for arterial blood gas analysis. "A utilization management intervention to reduce unnecessary testing in the coronary care unit", 9 September 2002, by Thomas J Wang et al, discloses a determination of whether a 3-part intervention in a coronary care unit could decrease utilization without affecting clinical outcomes. The settings of the ventilator may be changed depending on how the neonate is responding to treatment, with the response evaluated by measuring various parameters. The Arterial Blood Gas ("ABG") test is an important test that is conducted to measure key parameters for adjusting ventilator settings. However, the ABG test is both expensive to conduct and painful to administer; therefore, it is desirable to optimize the selection of the frequency at which it is performed.

This is achieved by a method as claimed in claim 1 and a system as claimed in claim 8.

Figure 1 shows an exemplary set of rules governing assisted ventilation of a neonate.

Figure 2 shows an exemplary method for selecting a frequency of ABG testing according to an exemplary embodiment.

Figure 3 shows an exemplary system for selecting a frequency of ABG testing according to an exemplary embodiment.

The exemplary embodiments may be further understood with reference to the following description of exemplary embodiments and the related appended drawings, wherein like elements are provided with the same reference numerals. Specifically, the exemplary embodiments relate to methods and systems for optimizing the selection of the frequency of arterial blood gas ("ABG") testing for neonatal intensive care patients.

Neonates (i.e., newborns) who are being treated in a neonatal intensive care unit ("NICU") for severe respiratory distress are typically treated with a ventilator and are continuously monitored using patient monitors, ventilator parameters, and various other tests. The results of this monitoring is parameters that are used to adjust the parameters of the ventilator. One important test is the ABG test, which is used to obtain values for partial pressure of oxygen ("PaO2") and partial pressure of carbon dioxide ("PaCO2"). However, the ABG test is both invasive, and therefore painful to the neonate, and expensive to administer. Therefore, it is highly desirable to perform ABG testing only at optimal time intervals, in order to minimize both the infliction of pain on the neonate and the cost of the testing.

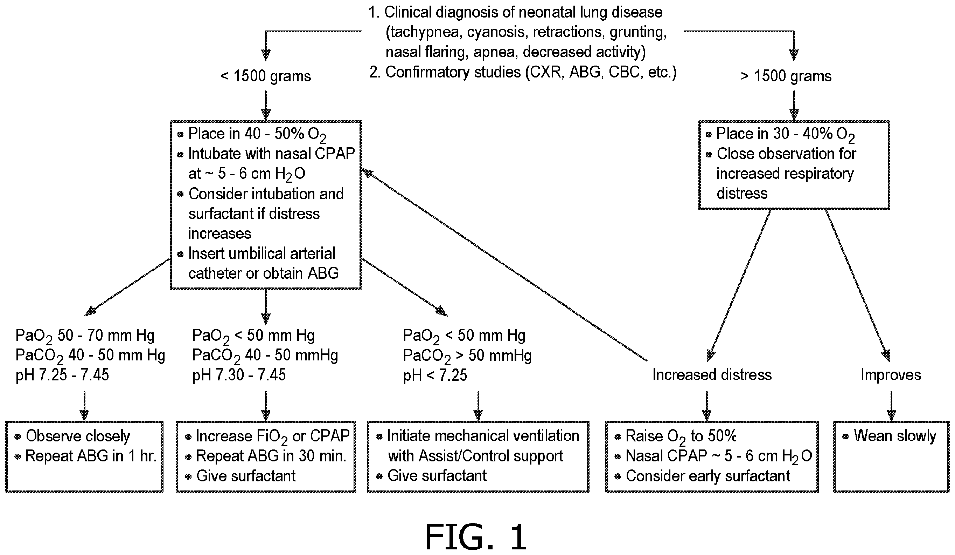

Typically, based on the results of the most recent ABG test, the settings of the ventilator may be adjusted and the time for the next ABG test may be selected. Figure 1 illustrates an exemplary set of rules governing such selections, as defined in "Assisted Ventilation of the Neonate", by Jay P. Goldsmith and Edward H. Karotkin, Fifth Edition, 2010. It will be apparent to those of skill in the art that this set of rules defines the subsequent treatment of the neonate, including adjusting the ventilator settings (or leaving the settings unchanged) and determining a time to repeat the ABG test.

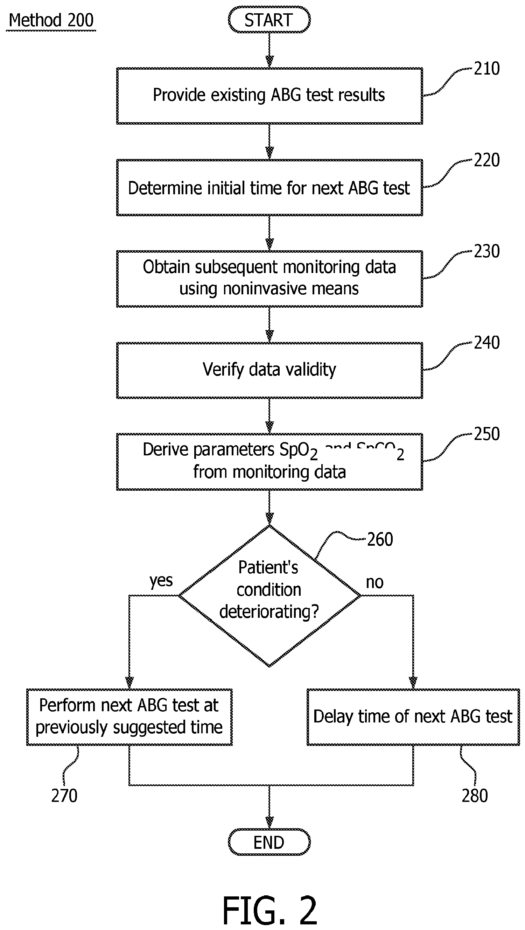

Figure 2 illustrates an exemplary method 200 for optimizing the determination of the time to conduct a subsequent ABG test. In step 210, the results of an existing ABG test are provided. This may entail the consideration of the results of a current ABG test or, alternately, the retrieval of the results of a previously-performed test, such as from a medical records database or any other suitable storage medium. In step 220, an initial time for a next ABG test is determined based on the results of the existing ABG test using known methods, such as the methodology outlined in Figure 1.

In step 230, subsequent monitoring data for the neonatal patient is obtained by noninvasive means. This step may include testing blood oxygen saturation ("SpO2") using a pulse oximeter and testing for end-tidal carbon dioxide ("EtCO2") using the ventilator or other another capnographic technique. These values may also be obtained using transcutaneous monitoring (e.g., tcO2 and tcCO2) or an SpO2 camera. The patient may also be monitored using a camera (e.g., an analog or digital video camera or camera capturing a series of still images), which may detect changes in the skin tone of the patient.

In step 240, the validity of the data obtained in step 230 is verified. This step may be necessary because the data may not be reliable under certain conditions (e.g., depending on the type of monitoring used to obtain the data, or on the patient's condition, such as apnea of prematurity). For example, if SpO2 is one of the types of patient monitoring data obtained in step 230, tracings of SpO2 may be used to determine the validity. Alternately, a reliable value for SpO2 may be obtained using Signal Extraction Technology.

In step 250, the parameters PaO2 and PaCO2 are derived from the patient data obtained in step 230 and validated in step 240. Those of skill in the art will understand that there are various means for performing such derivation. In one exemplary embodiment, PaO2 may be determined based on SpO2 using the expression:

$$PaO2 = \left(0.03\right) \cdot e^{0.08(SpO2)}$$

In the above expression, PaO2 is expressed in Torr. Additionally, PaCO2 may be determined based on EtCO2, as described in "Relationship Between Arterial Carbon Dioxide And End-Tidal Carbon Dioxide When A Nasal Sampling Port Is Used", by Stephen E. McNulty et al., Journal of Clinical Monitoring, April 1990.

In step 260, it is determined whether the patient's condition is deteriorating. This determination may be made by comparing the derived values of PaO2 and PaCO2 to their values as measured during the most recent ABG test, and by comparing them to prescribed ranges (e.g., as illustrated in Figure 1). The determination of the patient's condition may also involve the examination of camera-recorded still or video imagery; for example, neonates may become pale when oxygenation is insufficient. Alternately, the patient can be visually observed by a neonatologist, who may then indicate whether the patient has become pale.

If the patient's condition is determined to be deteriorating in step 260, then, in step 270, the next ABG test is performed at or within the time suggested by the results of the previous ABG test. In contrast, if, in step 260, the patient's condition is determined not to be deteriorating, then, in step 280, the time of the next ABG test is delayed to the time that may be suggested by the method illustrated in Figure 1. After either step 270 or step 280, the method 200 terminates. However, those of skill in the art will understand that the input measurements described above may be obtained continually and noninvasively, and so the method 200 may be continuously performed between ABG tests in order to provide up-to-date monitoring of the patient's condition In one embodiment, the determination of whether the patient's condition is deteriorating, and the resulting recommendation of the time for the next ABG test, may be made by a clinical decision support system, as described hereinafter.

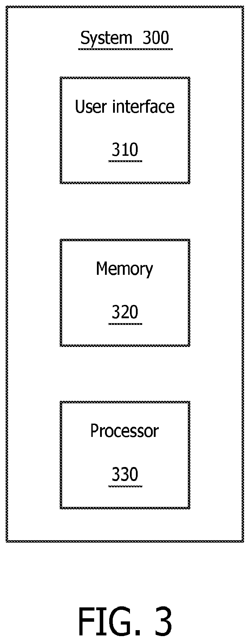

Figure 3 illustrates an exemplary system 300 for determining an optimal time for a next ABG test using a method such as the method 200. The system 300 includes a user interface 310, which may receive input data regarding ABG tests and other patient monitoring data as described above. In one embodiment, the user interface may be coupled directly to patient monitoring information in order to simplify the data communication process. The system 300 additionally includes a memory 320 storing a program embodying a method such as the method 200, and a processor 330 performing the method in order to provide output as described above. The output may be provided by means of the user interface 310.

The exemplary embodiments enable the timing of the ABG test to be optimized. As described above, this may be accomplished automatically using a system such as a clinical decision support system that may receive input from a clinician and output a recommended time. As a result, the costs of administering a series of ABG tests may be minimized, and neonatal patients may be spared from more invasive procedures than are necessary.

It is noted that the claims may include reference signs/numerals in accordance with PCT Rule 6.2(b). However, the present claims should not be considered to be limited to the exemplary embodiments corresponding to the reference signs/numerals.

It will be apparent to those skilled in the art that various modifications may be made to the exemplary embodiments, without departing from the scope of the invention. Thus, it is intended that the present invention cover modifications and variations of this invention provided they come within the scope of the appended claims.

## Claims

1. A computer-implemented method, comprising:

   receiving (210) previous arterial blood gas, ABG, test results for a neonate;
   determining (220) an initial time for a next ABG test for the neonate based on the previous ABG test results and a set of rules defining subsequent treatment of the neonate;
   receiving (230) monitoring data for the neonate;
   determining (280) a modified time for a next ABG test based on the initial time for the next ABG test and the neonate monitoring data; and
   deriving (250) neonate oxygenation parameters based on the monitoring data,
   wherein the modified time for the next ABG test is determined based on the initial time for the next ABG test and the neonate oxygenation parameters;
   wherein determining (280) the modified time for the next ABG test comprises delaying the next ABG test if the monitoring data indicates that a condition of the neonate has not deteriorated; and
   wherein determining (280) the modified time for the next ABG test comprises accelerating the next ABG test if the monitoring data indicates that a condition of the neonate has deteriorated.

2. The method of claim 1, wherein the neonate oxygenation parameters are PaO2 and PaCO2.

3. The method of claim2, wherein PaO2 is determined based on one of SpO2 and tcCO2.

4. The method of claim 3, wherein SpO2 is determined based on the results of a pulse oximeter test.

5. The method of claim 2, wherein PaCO2 is determined based on one of EtCO2 and tcCO2.

6. The method of claim 1, further comprising:

receiving an image of the neonate,
wherein the modified time for the next ABG test is further determined based on the image of the neonate.

7. The method of claim 6, wherein the determination is based on whether the image indicates that the neonate is paler than a previous image.

8. A system, comprising:

a patient monitor (310) detecting monitoring data for a neonate;
a memory (320) storing previous arterial blood gas, ABG, test results for the neonate and an initial time for a next ABG test determined based on the previous ABG test results and a set of rules defining subsequent treatment of the neonate; and
a processor (330) determining a modified time for a next ABG test based on the initial time for the next ABG test and the neonate monitoring data;
wherein the processor (330) further derives neonate oxygenation parameters based on the monitoring data, wherein the modified time for the next ABG test is determined based on the initial time for the next ABG test and the neonate oxygenation parameters;
wherein the processor (330) determines the modified time for the next ABG test comprises delaying the next ABG test if the monitoring data indicates that a condition of the neonate has not deteriorated; and
wherein the processor (330) determines the modified time for the next ABG test comprises accelerating the next ABG test if the monitoring data indicates that a condition of the neonate has deteriorated.

9. The system of claim 8, wherein the neonate oxygenation parameters are $PaO_2$ and $PaCO_2$.

10. The system of claim 9, wherein $PaO_2$ is determined based on one of $SpO_2$ and $tcCO_2$.

11. The system of claim 10, wherein $SpO_2$ is determined based on the results of a pulse oximeter test.

12. The system of claim 9, wherein $PaCO_2$ is determined based on one of $EtCO_2$ and $tcCO_2$.

13. The system of claim 8, wherein the processor (330) receives an image of the neonate, wherein the modified time for the next ABG test is further determined based on the image of the neonate.

14. The system of claim 13, wherein the determination is based on whether the image indicates that the neonate is paler than a previous image.

**Patentansprüche**

1. Computerimplementiertes Verfahren, umfassend:

Empfangen (210) voriger Arterienblutgas-, ABG, Testergebnisse für ein Neugeborenes;
Ermitteln (220) einer Anfangszeit für einen nächsten ABG-Test für das Neugeborene, basierend auf den vorigen ABG-Testergebnissen und einem Satz von Regeln, die nachfolgende Behandlung des Neugeborenen definieren;
Empfangen (230) von Überwachungsdaten für das Neugeborene;
Ermitteln (280) einer modifizierten Zeit für einen nächsten ABG-Test, basierend auf der Anfangszeit für den nächsten ABG-Test und die Überwachungsdaten des Neugeborenen; und
Ableiten (250) von Sauerstoffanreicherungsparametern des Neugeborenen, basierend auf den Überwachungsdaten,
wobei die modifizierte Zeit für den nächsten ABG-Test basierend auf der Anfangszeit für den nächsten ABG-Test und den Sauerstoffanreicherungsparametern des Neugeborenen ermittelt wird;
wobei Ermitteln (280) der modifizierten Zeit für den nächsten ABG-Test Verzögern des nächsten ABG-Tests umfasst, falls die Überwachungsdaten anzeigen, dass ein Zustand des Neugeborenen sich nicht verschlechtert hat; und
wobei Ermitteln (280) der modifizierten Zeit für den nächsten ABG-Test Beschleunigen des nächsten ABG-Tests umfasst, falls die Überwachungsdaten anzeigen, dass sich ein Zustand des Neugeborenen verschlechtert hat.

2. Verfahren nach Anspruch 1, wobei die Sauerstoffanreicherungsparameter des Neugeborenen $PaO_2$ und $PaCO_2$ sind.

3. Verfahren nach Anspruch 2, wobei $PaO_2$ basierend auf einem von $SpO_2$ und $tcCO_2$ ermittelt wird.

4. Verfahren nach Anspruch 3, wobei $SpO_2$ basierend auf den Ergebnissen eines Pulsoximetertests ermittelt wird.

5. Verfahren nach Anspruch 2, wobei $PaCO_2$ basierend auf einem von $EtCO_2$ und $tcCO_2$ ermittelt wird.

6. Verfahren nach Anspruch 1, weiter umfassend:

Empfangen eines Bilds des Neugeborenen,

wobei die modifizierte Zeit für den nächsten ABG-Test weiter basierend auf dem Bild des Neugeborenen ermittelt wird.

7. Verfahren nach Anspruch 6, wobei die Ermittlung darauf basiert, ob das Bild anzeigt, dass das Neugeborene blasser als in einem vorigen Bild ist.

8. System, umfassend:

einen Patientenmonitor (310), der Überwachungsdaten für ein Neugeborenes erfasst; einen Speicher (320), der vorige Arterienblutgas-, ABG, Testergebnisse für das Neugeborene und eine Anfangszeit für einen nächsten ABG-Test, speichert, die basierend auf dem vorigen ABG-Testergebnissen und einem Regelsatz, der nachfolgende Behandlung des Neugeborenen definiert, ermittelt ist; und einen Prozessor (330), der eine modifizierte Zeit für einen nächsten ABG-Test basierend auf der Anfangszeit für den nächsten ABG-Test und den Überwachungsdaten des Neugeborenen ermittelt; wobei der Prozessor (330) weiter Sauerstoffanreicherungsparameter des Neugeborenen basierend auf den Überwachungsdaten ableitet, wobei die modifizierte Zeit für den nächsten ABG-Test basierend auf der Anfangszeit für den nächsten ABG-Test und den Sauerstoffanreicherungsparametern des Neugeborenen ermittelt wird; wobei, dass der Prozessor (330) die modifizierte Zeit für den nächsten ABG-Test ermittelt, umfasst, den nächsten ABG-Test zu verzögern, falls die Überwachungsdaten anzeigen, dass sich ein Zustand des Neugeborenen nicht verschlechtert hat; und wobei, dass der Prozessor (330) die modifizierte Zeit für den nächsten ABG-Test ermittelt, umfasst, den nächsten ABG-Test zu beschleunigen, falls die Überwachungsdaten anzeigen, dass sich ein Zustand des Neugeborenen verschlechtert hat.

9. System nach Anspruch 8, wobei die Sauerstoffanreicherungsparameter des Neugeborenen PaO2 und PaCO2 sind.

10. System nach Anspruch 9, wobei PaO2 basierend auf einem von SpO2 und tcCO2 ermittelt wird.

11. System nach Anspruch 10, wobei SPO2 basierend auf den Ergebnissen eines Pulsoximetertests ermittelt wird.

12. System nach Anspruch 9, wobei PaCO2 basierend auf einem von EtCO2 und tcCO2 ermittelt wird.

13. System nach Anspruch 8, wobei der Prozessor (330) ein Bild des Neugeborenen empfängt, wobei die modifizierte Zeit für den nächsten ABG-Test weiter basierend auf dem Bild des Neugeborenen ermittelt wird.

14. System nach Anspruch 13, wobei die Ermittlung darauf basiert, ob das Bild anzeigt, dass das Neugeborene blasser als in einem vorigen Bild ist.

**Revendications**

1. Procédé mis en oeuvre par ordinateur, comprenant :

la réception (210) de résultats de test précédent de gazométrie du sang artériel, ABG, pour un nouveau-né ; la détermination (220) d'un temps initial pour un test d'ABG suivant pour le nouveau-né sur la base des résultats de test d'ABG précédent et un ensemble de règles définissant un traitement ultérieur du nouveau-né ; la réception (230) de données de surveillance pour le nouveau-né ; la détermination (280) d'un temps modifié pour un test d'ABG suivant sur la base du temps initial pour le test d'ABG suivant et des données de surveillance de nouveau-né ; et l'obtention (250) de paramètres d'oxygénation de nouveau-né sur la base des données de surveillance, dans lequel le temps modifié pour le test d'ABG suivant est déterminé sur la base du temps initial pour le test d'ABG suivant et des paramètres d'oxygénation de nouveau-né ; dans lequel la détermination (280) du temps modifié pour le test d'ABG suivant comprend le retardement du test d'ABG suivant si les données de surveillance indiquent qu'un état du nouveau-né ne s'est pas détérioré ; et dans lequel la détermination (280) du temps modifié pour le test d'ABG suivant comprend l'accélération du test d'ABG suivant si les données de surveillance indiquent qu'un état du nouveau-né s'est détérioré.

2. Procédé selon la revendication 1, dans lequel les paramètres d'oxygénation de nouveau-né sont PaO2 et PaCO2.

3. Procédé selon la revendication 2, dans lequel PaO2 est déterminé sur la base de l'un de SpO2 et tcCO2.

4. Procédé selon la revendication 3, dans lequel SpO2 est déterminé sur la base des résultats d'un test par oxymètre de pouls.

**5.** Procédé selon la revendication 2, dans lequel PaCO2 est déterminé sur la base de l'un d'EtCO2 et de tcCO2.

**6.** Procédé selon la revendication 1, comprenant en outre :

la réception d'une image du nouveau-né, dans lequel le temps modifié pour le test d'ABG suivant est en outre déterminé sur la base de l'image du nouveau-né.

**7.** Procédé selon la revendication 6, dans lequel la détermination est basée sur le fait que l'image indique que le nouveau-né est plus blême que sur une image précédente.

**8.** Système, comprenant :

un dispositif de surveillance de patient (310) détectant des données de surveillance pour un nouveau-né ;
une mémoire (320) stockant des résultats de test précédent de gazométrie du sang artériel, ABG, pour le nouveau-né et un temps initial pour un test d'ABG suivant déterminé sur la base des résultats de test d'ABG précédent et d'un ensemble de règles définissant un traitement ultérieur du nouveau-né ; et
un processeur (330) déterminant un temps modifié pour un test d'ABG suivant sur la base du temps initial pour le test d'ABG suivant et des données de surveillance de nouveau-né ;
dans lequel le processeur (330) obtient en outre des paramètres d'oxygénation de nouveau-né sur la base des données de surveillance, dans lequel le temps modifié pour le test d'ABG suivant est déterminé sur la base du temps initial pour le test d'ABG suivant et des paramètres d'oxygénation de nouveau-né ;
dans lequel le processeur (330) détermine que le temps modifié pour le test d'ABG suivant comprend le retardement du test d'ABG suivant si les données de surveillance indiquent qu'un état du nouveau-né ne s'est pas détérioré ; et
dans lequel le processeur (330) détermine que le temps modifié pour le test d'ABG suivant comprend l'avancement du test d'ABG suivant si les données de surveillance indiquent qu'un état du nouveau-né s'est détérioré.

**9.** Système selon la revendication 8, dans lequel les paramètres d'oxygénation de nouveau-né sont PaO2 et PaCO2.

**10.** Système selon la revendication 9, dans lequel PaO2 est déterminé sur la base de l'un de SpO2 et de tcCO2.

**11.** Système selon la revendication 10, dans lequel SpO2 est déterminé sur la base des résultats d'un test par oxymètre de pouls.

**12.** Système selon la revendication 9, dans lequel PaCO2 est déterminé sur la base de l'un d'EtCO2 et de tcCO2.

**13.** Système selon la revendication 8, dans lequel le processeur (330) reçoit une image du nouveau-né, dans lequel le temps modifié pour le test d'ABG suivant est en outre déterminé sur la base de l'image du nouveau-né.

**14.** Système selon la revendication 13, dans lequel la détermination est basée sur le fait que l'image indique que le nouveau-né est plus blême qu'une image précédente.

1. Clinical diagnosis of neonatal lung disease (tachypnea, cyanosis, retractions, grunting, nasal flaring, apnea, decreased activity)
2. Confirmatory studies (CXR, ABG, CBC, etc.)

< 1500 grams

> 1500 grams

- Place in 40 - 50% $O_2$
- Intubate with nasal CPAP at ~ 5 - 6 cm $H_2O$
- Consider intubation and surfactant if distress increases
- Insert umbilical arterial catheter or obtain ABG

- Place in 30 - 40% $O_2$
- Close observation for increased respiratory distress

$PaO_2$ 50 - 70 mm Hg
$PaCO_2$ 40 - 50 mm Hg
pH 7.25 - 7.45

$PaO_2$ < 50 mm Hg
$PaCO_2$ 40 - 50 mmHg
pH 7.30 - 7.45

$PaO_2$ < 50 mm Hg
$PaCO_2$ > 50 mmHg
pH < 7.25

Increased distress

Improves

- Observe closely
- Repeat ABG in 1 hr.

- Increase $FiO_2$ or CPAP
- Repeat ABG in 30 min.
- Give surfactant

- Initiate mechanical ventilation with Assist/Control support
- Give surfactant

- Raise $O_2$ to 50%
- Nasal CPAP ~ 5 - 6 cm $H_2O$
- Consider early surfactant

- Wean slowly

# FIG. 1

EP 2 856 371 B1

Method 200

START

Provide existing ABG test results — 210

Determine initial time for next ABG test — 220

Obtain subsequent monitoring data using noninvasive means — 230

Verify data validity — 240

Derive parameters $SpO_2$ and $SpCO_2$ from monitoring data — 250

Patient's condition deteriorating? — 260

yes          no

Perform next ABG test at previously suggested time

270

Delay time of next ABG test

280

END

# FIG. 2

System  300

User interface

310

Memory

320

Processor

330

# FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PAOLO MERLANI.** *Quality Improvement Report - Linking guideline to regular feedback to increase appropriate requests for clinical tests: blood gas analysis in intensive care,* 15 September 2001
- **THOMAS J WANG.** *A utilization management intervention to reduce unnecessary testing in the coronary care unit,* 09 September 2002
- **JAY P. GOLDSMITH ; EDWARD H. KAROTKIN.** Assisted Ventilation of the Neonate. 2010
- Relationship Between Arterial Carbon Dioxide And End-Tidal Carbon Dioxide When A Nasal Sampling Port Is Used. **STEPHEN E. MCNULTY et al.** Journal of Clinical Monitoring. April 1990